# EUROPEAN PATENT APPLICATION

(11) **EP 3 053 538 A1**
(43) Date of publication of application: **10.08.2016**
(21) Application number: 14850779.1
(22) Date of filing: 30.09.2014
(51) Int. Cl.: A61B 18/20, A61N 5/067

(54) **FRACTIONAL LASER SURGICAL EQUIPMENT HAVING MULTIPLE PURPOSES INCLUDING TREATMENT OF VAGINA**

(30) Priority: 02.10.2013 KR 20130117820
(71) Applicant: Senbitec Co., Ltd., Gunpo-si, Gyeonggi-do 435-862 (KR)
(72) Inventor: YOON, Ki Joon, Anyang-si Gyeonggi-do 430-010 (KR)
(74) Representative: Isarpatent
(86) International application number: PCT/KR2014/009172
(87) International publication number: WO 2015/050358

(57) **Abstract**

The present invention relates to fractional laser surgical equipment having multiple purposes including treatment of the vagina, which can treat various vaginal diseases by being inserted into the vagina of a woman and irradiate lasers at normal skin, and comprises: a laser controller in which a laser supply portion is formed at one end of a main body having a space therein and a laser emission hole is formed at the other end of the main body; and a laser irradiation portion formed to enable attachment to or detachment from the laser controller and having a pair of guide portions formed at a part making contact with the affected part such that the laser is irradiated between the guide portions. The fractional laser surgical equipment having multiple purposes including treatment of the vagina enables accurate laser targeting of the affected part, can prevent various side effects such as repetitive laser irradiation, can wash in advance and antibacterially treat a plurality of insertion portions so as to be more sanitary since the insertion portions are attachable and detachable, and can reduce the time for operation preparation.

## Description

### [Technical Field]

The present invention relates to fractional laser surgical equipment having multiple purposes including treatment of the vagina, which can treat various vaginal diseases by being inserted into the vagina of a woman and irradiate lasers at normal skin.

### [Background Art]

Lasers have characteristics in that lasers having single wavelengths and have one color according to raw materials for generating energy (monochromaticity), laser light is not spread in all directions but has uniform directivity (collimation) and phases and wavelengths of lasers are temporally and spatially coherent. In addition, a laser shows strong brightness temporally, and energy of the laser can be concentrated on a part to be treated. When the energy is applied to a tissue formed of moisture, moisture evaporates instantaneously, and the tissue is vaporized and is destroyed. This principle can be used in surgery. A tissue can be cut by lasers instead of a scalpel and can be evaporated such that a lesion is removed.

Advantages of laser surgery are that, in laser surgery, surgery having no bleeding can be performed, a lesion can be accurately removed and a minute part can be operated on. In addition, laser surgery is better than a general surgery method in that inflammation after operation is light and thus there is less pain and less scarring, and a hospitalization period is short or not required.

Meanwhile, in fractional lasers, a single laser pulse is divided into a plurality of fractional laser beams so that many fine laser beams are irradiated. Thus, a coverage in which lasers are irradiated onto skin is wide and an operation time is reduced, vitality of fibroblasts is increased in a tissue into which lasers permeate, and because regeneration of collagen is promoted, proliferation of stem cells of the outer skin is induced so that movement of peripheral undamaged cells is induced and the skin can be regenerated.

Unlike the past, these days, laser surgery on inner walls of a vagina, such as a vaginoplasty of a woman, has been actively performed. This is because; due to pregnancy and childbirth of women, the muscle of the vagina sags or becomes loose, which results in a symptom, such as urinary incontinence or a sexual disorder.

Surgery on the inner walls of a vagina is performed by inserting laser surgical equipment into the vagina due to characteristics of the affected part. Since, in the related art, an operator (a surgeon) performs an operation by inserting the laser surgical equipment into a vagina and turning the laser surgical equipment by hand so that lasers are irradiated onto the coverage of the affected part. It is difficult to perform an accurate operation on the affected part. In addition, various side effects such as repetitive laser irradiation onto a section of the affected part occur.

In addition, in the conventional laser surgical equipment, a laser generating unit and guide portions inserted into a vagina are integrally configured. Thus, the laser generating unit and the guide portions should be washed one by one after operation, and thus it takes much time.

### [Disclosure]

### [Technical Problem]

The present invention is directed to providing fractional laser surgical equipment having multiple purposes including treatment of the vagina, whose insertion portion can be accurately rotated by a stepping motor so that accurate laser targeting of an affected part can be controlled and various side effects such as repetitive laser irradiation can be prevented and in which a plurality of insertion portions can be washed in advance and antibacterially treated so as to be more sanitary since the insertion portion is attachable and detachable and the time for operation preparation can be reduced.

One aspect of the present invention provides fractional laser surgical equipment having multiple purposes including treatment of the vagina, including: a laser controller in which a laser supply portion is formed at one end of a main body having a space therein and a laser emission hole is formed at the other end of the main body; and a laser irradiation portion formed to enable attachment to or detachment from the laser controller and having a pair of guide portions formed at a part making contact with an affected part such that a laser is irradiated between the guide portions.

The laser irradiation portion may include: a casing whose one side is attached to or detached from the laser controller, in which a stepping motor is embedded and which is embedded in the laser irradiation portion so that a coupling plate rotated by the stepping motor is exposed to the other side of the casing; a stepping module in which a focusing lens is embedded in a position of the casing facing the laser emission hole; and an insertion portion whose one side is coupled to the coupling plate, which has hollow portions formed therein and guide portions formed at the other side of the insertion portion so that a reflector is configured at distal ends of the guide portions.

In the stepping module, the coupling plate may interlock with a guide gear having a hollow portion therein, a stepping gear that interlocks with the stepping motor may be configured to be engaged with the guide gear, and hollow portions may be formed in positions of the coupling plate and the guide gear that face the focusing lens.

The guide portions may include a pair of bar-shaped frames in the center of the insertion portion and may be bent so that inclined portions are formed at distal ends of each frame, and the reflector may be fixed to the inclined portions.
A recognizing portion may be configured in the stepping module, and a recognizing portion connector may be configured in the insertion portion so that an electrical connection is made.

The recognizing portion may recognize one or more among a rotation quantity, a usage time, and a laser irradiation quantity of the insertion portion.

The laser irradiation portion may be a fixed module whose one side is fastened to the laser controller, in which a hollow portion, through which a laser emitted from the laser controller passes, is formed, and a focusing lens is configured in the hollow portion and which includes guide portions at the other side of the laser irradiation portion.

The guide portions having bar shapes may include a pair of ⊏-shaped frames.

### [Advantageous Effects]

As described above, in the fractional laser surgical equipment having multiple purposes including treatment of the vagina according to the present invention, a laser controller, a stepping module, and an insertion portion is coupled to one another to be attachable and detachable, and accurate rotational control of the insertion portion can be performed by the stepping module so that accurate laser targeting of an affected part can be performed and various side effects such as repetitive laser irradiation can be prevented.

In addition, since the insertion portion is attachable and detachable, a plurality of insertion portions can be washed in advance and antibacterially treated so as to be more sanitary, and the time for operation preparation can be reduced.

### [Description of Drawings]

FIG. 1 is a combined perspective view of fractional laser surgical equipment having multiple purposes including treatment of the vagina according to an embodiment of the present invention;
FIG. 2 is an exploded perspective view of the fractional laser surgical equipment having multiple purposes including treatment of the vagina according to the current embodiment;
FIG. 3 is a view illustrating a coverage of irradiation of lasers controlled as an insertion portion according to the current embodiment is rotated; and
FIG. 4 is an exploded perspective view of another embodiment of the present invention.

### [Modes of the Invention]

Hereinafter, a configuration and operation of the present invention will be described in more detail with reference to the attached drawings. In the description of the present invention, the terminology or words used in the present specification and the claims should be interpreted as a meaning and a concept that correspond to the technical spirit of the present invention based on the principle that the inventor can properly define the concept of terms so as to describe his/her own invention in the best manner.

Fractional laser surgical equipment having multiple purposes including treatment of the vagina according to the present invention that is equipment inserted into the vagina of a woman and irradiates lasers onto an affected part of inner walls of the vagina while rotating fully automatically with respect to various vaginal diseases (colpoxerosis, urinary incontinence, vaginal contraction improvement, and sexual disorder improvement), includes a laser controller 10 and laser irradiation portions 20 and 40.

Meanwhile, 'front' throughout the present specification is defined as a part to be treated of a patient, i.e., a direction from the laser controller 10 to the laser irradiation portions 20 and 40.

First, the laser controller 10 according to the present invention is configured to emit a laser onto the laser irradiation portion 20 by receiving laser beams from an additional laser generator (not shown) and setting a coverage of irradiation of the laser beams to correspond to a size and shape of an affected part, and includes a main body 100, a laser supply portion 120, a two-axis scanning mirror 130, and a laser emission hole 140.

In detail, the laser controller 10 is configured so that the laser supply portion 120 is formed at one end of the main body 100 having a space therein and the laser emission hole 140 is formed at the other end of the main body 100, and the laser controller 10 is formed to enable attachment to or detachment from the laser irradiation portion 20.

In addition, the laser irradiation portion 20 is configured to include a pair of guide portions 320 and 420 so as to maintain a predetermined distance with skin of an object onto which the laser is to be irradiated, and to irradiate the laser between the guide portions 320 and 420.

Meanwhile, in fractional lasers, a single laser pulse is divided into a plurality of fractional laser beams so that many fine laser beams are irradiated to the skin. Thus, a coverage in which lasers are irradiated onto skin is wide and an operation time is reduced, vitality of fibroblasts is increased in a tissue into which lasers permeate, and because regeneration of collagen is promoted, proliferation of stem cells of the outer skin is induced so that movement of peripheral undamaged cells is induced and the skin can be regenerated.

In the current embodiment, the fractional laser surgical equipment having multiple purposes including treatment of the vagina is inserted into a vagina so as to perform an operation. When the fractional laser surgical equipment having multiple purposes including treatment of the vagina is configured so that the laser controller 10 and the laser irradiation portion 20 are provided to be attachable and detachable, the laser irradiation portion 20 includes a stepping module 200 and an insertion portion 300. Thus, when the fractional laser surgical equipment having multiple purposes including treatment of the vagina is inserted into the vagina of a patient so as to perform an operation, replacement of the insertion portion 300 is possible so that sanitation and cleanliness can be maintained.

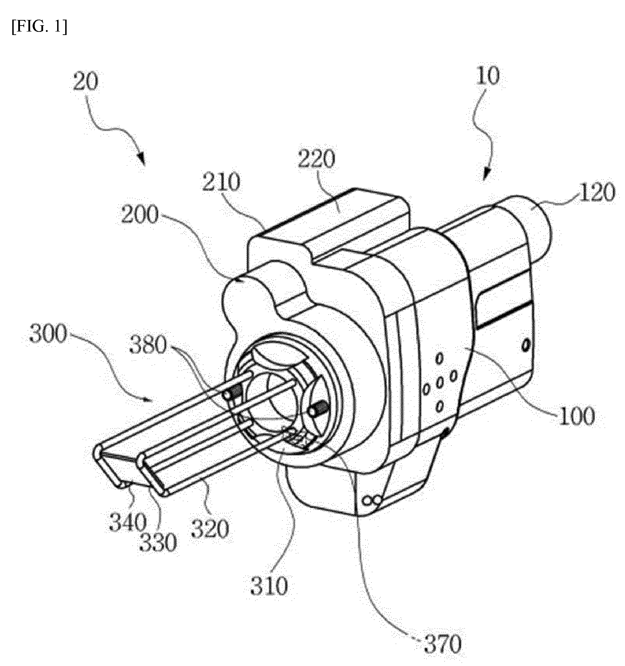

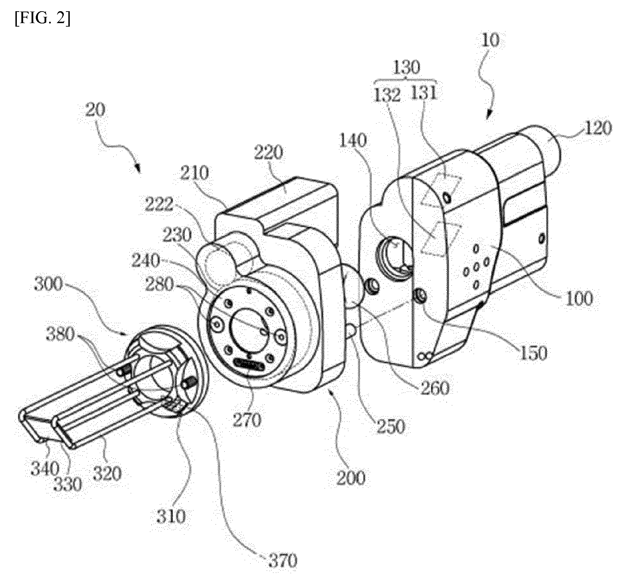

In detail with reference to FIGS. 1 and 2, FIGS. 1 and 2 are a combined perspective view and an exploded perspective view of the fractional laser surgical equipment having multiple purposes including treatment of the vagina according to the current embodiment and show a combined structure of the laser controller 10, the stepping module 200, and the insertion portion 300 and an operational relationship therebetween.

First, the laser controller 10 according to the current embodiment is configured to irradiate a laser onto the stepping module 200 and the insertion portion 300 that will be described in detail later by receiving laser beams from an additional laser generator (not shown) and setting a coverage of irradiation of laser beams to correspond to a size and shape of an affected part, and includes the main body 100, the laser supply portion 120, the two-axis scanning mirror 130, and the laser emission hole 140.

In detail, the laser controller 10 includes the main body 100 having a space therein, the two-axis scanning mirror 130 that is provided in the main body 100 and controls the coverage of irradiation of the laser beams, the laser supply portion 120 that is formed at one end of the main body 100 disposed so that the laser beams are irradiated onto a first scanning mirror 131 of the two-axis scanning mirror 130 and that receives the laser from the external laser generator (not shown), and the laser emission hole 140 that is formed at the other end of the main body 100 and emits the laser beams set by the two-axis scanning mirror 130.

First, the laser supply portion 120 having a shape of a tube with a hollow portion or a box shape is configured to connect the external laser generator (not shown) and the main body 100 so that the laser beams are supplied to the main body 100. In this case, one end of the laser supply portion 120 is coupled to the main body 100, and the other end of the laser supply portion 120 is connected to the laser generator.

Meanwhile, since the two-axis scanning mirror 130 is provided in the main body 100 of the laser controller 10, the two-axis scanning mirror 130 enables the first scanning mirror 131 and a second scanning mirror 132 to adjust an angle therebetween so that the coverage of irradiation of the laser beams is controlled. In the present invention, the structure of the conventional two-axis scanning mirror 130 is applied, and a detailed description of a principle thereof will be omitted.

The laser beams incident through the laser supply portion 120 formed at one end of the main body 100 are irradiated onto the first scanning mirror 131 provided in the main body 100, and the laser beams reflected from the first scanning mirror 131 are reflected from the second scanning mirror 132 and are emitted into the laser emission hole 140 formed at the other end of the main body 100.

In addition, one or more seating grooves 150 are formed in a side facing the front of the laser controller 10, i.e., a side in which the laser emission hole 140 is formed, and are coupled to seating protrusions 250 of the stepping module 200 which will be described later.

The stepping module 200 according to the current embodiment includes a stepping motor 220, a stepping gear 222, a guide gear 230, a focusing lens 260, and a coupling plate 240. The stepping module 200 is coupled to the insertion portion 300, which will be described in detail later, and controls the insertion portion 300 to be accurately rotated.

In detail, the stepping module 200 includes a casing 210 whose one side is attached to or detached from the laser controller 10, in which the stepping motor 220 is embedded and which is embedded in the stepping module 200 so that the coupling plate 240 rotated by the stepping motor 220 is exposed to the other side of the casing 210, and a focusing lens 260 is embedded in the casing 210 in a position facing the laser emission hole 140.

In addition, the coupling plate 240 interlocks with the guide gear 230 having a hollow portion therein, and the stepping gear 222 that interlocks with the stepping motor 220 is configured to be engaged with the guide gear 230, and hollow portions are formed in positions of the coupling plate 240 and the guide gear 230 that face the focusing lens 260.

Because a hollow portion, through which the laser beams controlled by the laser controller 10 pass in a forward direction, should be formed in the stepping module 200, the stepping module 200 includes the guide gear 230 having the hollow portion therein and the coupling plate 240 having the hollow portion therein and is disposed in front of the guide gear 230. Thus, the stepping module 200 has a configuration in which the laser beams emitted from the laser controller 10 may pass through the hollow portions.

The guide gear 230 that interlocks with the coupling plate 240 so as to provide a rotational force to the coupling plate 240 is disposed to be engaged with the stepping gear 222, and the stepping gear 222 is configured to interlock with a rotating shaft of the stepping motor 220. In this case, the stepping motor 220 that is a motor rotating at an angle proportional to a predetermined number of pulses by assigning an order to the pulses in a step state, enables accurate rotational control of the coupling plate 240.

In addition, a hollow portion is formed in a position of the casing 210 that faces the laser emission hole 140, and the focusing lens 260 is embedded in the hollow portion. The focusing lens 260 may be disposed to be focused on a reflector 340 that is one configuration of the insertion portion 300, which will be described in detail later.

The insertion portion 300 according to the current embodiment is configured to be directly inserted into the vagina of a patient so that the laser is irradiated onto an affected part, and includes a motor coupling portion 310, the guide portion 320, and the reflector 340.

First, the motor coupling portion 310 having a shape of a disc with a hollow portion is configured to be coupled to the coupling plate 240 that is one configuration of the stepping module 200, as described above, so that the insertion portion 300 is fixed to be rotatable and the laser passes in the forward direction through the hollow portion formed therein. In this case, the coupling plate 240 is coupled to the insertion portion 300 to interlock therewith and controls a rotation of the insertion portion 300.

The guide portions 320 include a pair of bar-shaped frames in the center of the insertion portion 300 and are bent so that inclined portions 330 are formed at distal ends of the frames, and the reflector 340 is fixed to the inclined portions 330.

This is because the configuration of the guide portions 320 including bar-shaped frames enables a part making contact with skin to be reduced when the insertion portion is inserted into a vagina so that damage of the skin due to the insertion portion may be prevented, and the configuration of the guide portions 320 including a pair of frames enables a space to be secured when the laser irradiated through the laser controller 10 is irradiated onto the reflector 340 so that a course of the laser is prevented from being blocked by a contraction of the vagina, and thus inner walls of the vagina not related to treatment are prevented from being damaged by the laser.

The reflector 340 according to the current embodiment refracts forward-irradiated laser beams sideward so that the laser is irradiated onto the inner walls of the vagina. The reflector 340 refracts an irradiation angle of the forward-irradiated laser beams through the laser controller 10 and the stepping module 200 vertically so that the laser is irradiated onto the inner walls of the vagina that is the affected part.

Preferably, an inclination angle of the reflector 340 may be 45° based on a progression direction of the laser beams. However, the inclination angle of the reflector 340 may be an angle at which the forward laser is refracted and thus can be irradiated onto the inner walls of the vagina.

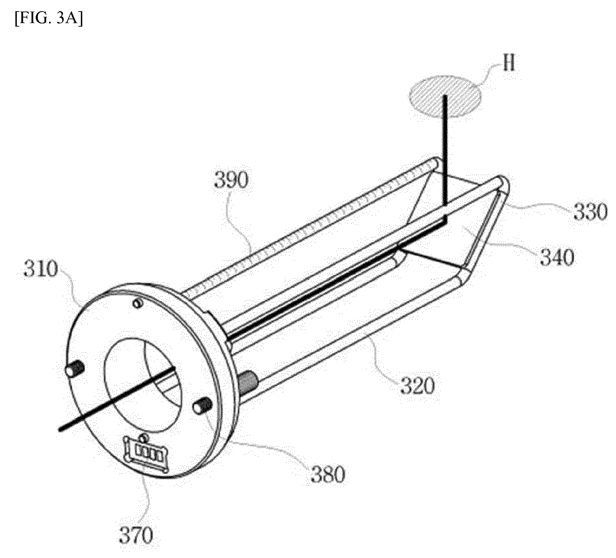

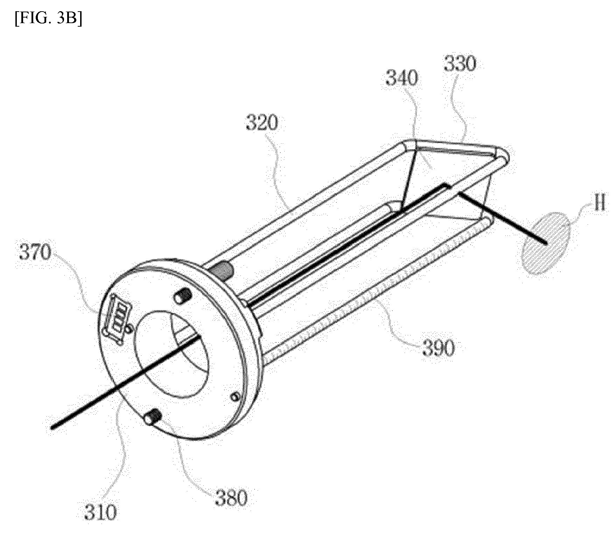

FIGS. 3A and 3B illustrate the coverage of irradiation of the laser controlled as the insertion portion 300 is rotated. When an operator inserts the fractional laser surgical equipment having multiple purposes including treatment of the vagina into the vagina of a patient, the operator can perform an operation while the insertion portion 300 is automatically rotated to correspond to a preset coverage of an affected part H.

Meanwhile, gradations 390 are marked at one side of each guide portion 320 so that the operator can insert the insertion portion 300 while seeing the gradations 390 of a guide to a preset depth value of the affected part H, and thus a more accurate operation can be performed.

Furthermore, the coupling plate 240 is coupled to the insertion portion 300, and thus controls the rotation of the insertion portion 300. A coupling bolt 380 may be used as a coupling unit of the coupling plate 240 and the insertion portion 300, and the coupling bolt 380 is configured to be fastened to a plurality of bolt grooves 280 formed in the coupling plate 240. However, the present invention is not limited thereto, and a fastening unit that enables the coupling plate 240 and the insertion portion 300 to be integrally rotatable may be used.

A recognizing portion 270 is further configured in the coupling plate 240 to be in contact with a recognizing portion connector 370 configured in the insertion portion 300 when the insertion portion 300 is fastened so that whether the insertion portion 300 is accurately fastened to the coupling plate 240 may be checked, and thus a malfunction of a device can be prevented. In addition, although a rotation quantity, a usage time and a laser irradiation quantity of the insertion portion 300 are not shown, they are marked or recognized through an additional monitor or speaker so that a set replacement period of the insertion portion 300 may be detected and thus sanitation and cleanliness can be maintained by preventing a long-term use for one patient or a replacement of the insertion portion whenever a patient changes.

In addition, although not shown, a waterproof coating and an antibacterial coating are performed on the surface of each guide portion 320 so that they may be more sanitarily used.

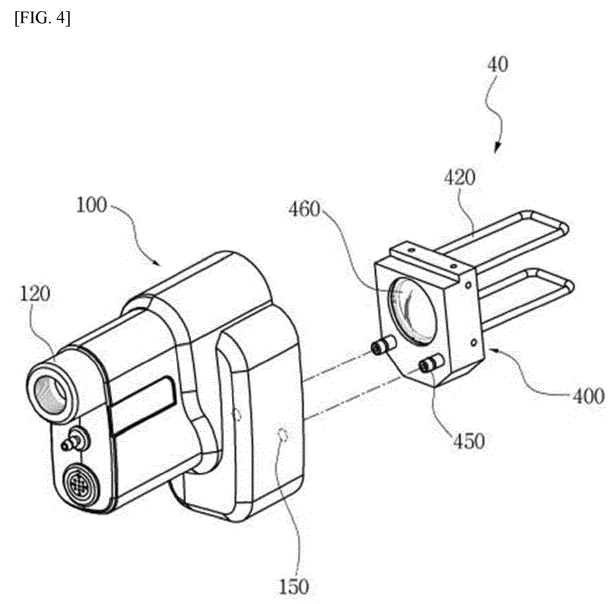

FIG. 4 is an exploded perspective view of another embodiment of the present invention. In the current embodiment, the laser controller 10 and the laser irradiation portion 40 are configured to be attachable and detachable so that lasers are irradiated at normal skin in addition to the vagina of a woman using the fractional laser surgical equipment having multiple purposes including treatment of the vagina. The laser irradiation portion 40 includes a fixed module 400. Thus, in the current embodiment, a plurality of fixed modules 400 having various treatment effects are configured to be assembled with and disassembled from one laser controller 10.

Generally, high-priced laser surgical equipment should be purchased in a hospital, in particular, to be suitable for various sizes or shapes according to uses depending on treatment purposes or a part to be treated. On the other hand, laser surgical equipment capable of replacing a module suggested in the current embodiment is used so that cost-reduction effects can be attained.

In the current embodiment, a skin treatment fixed module 400 among the plurality of fixed modules 400 having various shapes and sizes has been suggested. It should be obvious that the present invention is not limited thereto.

The laser controller 10 is fastened to one side of the fixed module 400 in the current embodiment, and a hollow portion, through which the laser emitted from the laser controller 10 passes, is formed in the fixed module 400, and the focusing lens 460 is configured in the hollow portion, and the fixed module 400 includes guide portions 420 at the other side thereof.

Meanwhile, when the guide portions 420 having bar shapes include a pair of ⊏-shaped frames and an operation is performed, the laser is irradiated in close contact with skin so that a predetermined distance between the laser and the skin can be maintained and a laser surgical operation can be effectively performed.

In addition, the seating protrusions 450 are formed at one side of the fixed module 400 in positions corresponding to the seating grooves 150, which are one configuration of the laser controller 10, to be attached to or detached from the laser controller 10.

While the invention has been shown and described with reference to certain exemplary embodiments thereof, it should be understood by those skilled in the art that various changes in form and details may be made therein without departing from the spirit and scope of the invention as defined by the appended claims.

**[Explanation of Reference Numerals]**

| | |
|---|---|
| 10 : laser controller | 20,40 : laser irradiation portion |
| 100 : main body | 120 : laser supply portion |
| 130 : two-axis scanning mirror | 140 : laser emission hole |
| 150 : seating grooves | 200 : stepping module |
| 210 : casing | 220 : stepping motor |
| 230 : guide gear | 240 : coupling plate |
| 300 : insertion portion | 310 : motor coupling portion |
| 320 : guide portions | 330 : inclined portions |
| 340 : reflector | 400 : fixed module |

## Claims

1. Fractional laser surgical equipment having multiple purposes including treatment of the vagina, comprising:
a laser controller in which a laser supply portion is formed at one end of a main body having a space therein and a laser emission hole is formed at the other end of the main body; and
a laser irradiation portion formed to enable attachment to or detachment from the laser controller and having a pair of guide portions formed at a part making contact with an affected part such that a laser is irradiated between the guide portions.

2. The fractional laser surgical equipment of claim 1, wherein the laser irradiation portion comprises:
a casing whose one side is attached to or detached from the laser controller, in which a stepping motor is embedded and which is embedded in the laser irradiation portion so that a coupling plate rotated by the stepping motor is exposed to the other side of the casing;
a stepping module in which a focusing lens is embedded in a position of the casing facing the laser emission hole; and
an insertion portion whose one side is coupled to the coupling plate, which has hollow portions formed therein and guide portions formed at the other side of the insertion portion so that a reflector is configured at distal ends of the guide portions.

3. The fractional laser surgical equipment of claim 2, wherein, in the stepping module, the coupling plate interlocks with a guide gear having a hollow portion therein, a stepping gear that interlocks with the stepping motor is configured to be engaged with the guide gear, and hollow portions are formed in positions of the coupling plate and the guide gear that face the focusing lens.

4. The fractional laser surgical equipment of claim 2, wherein the guide portions comprise a pair of bar-shaped frames in the center of the insertion portion and are bent so that inclined portions are formed at distal ends of each frame, and the reflector is fixed to the inclined portions.

5. The fractional laser surgical equipment of claim 2, wherein a recognition portion is configured in the stepping module, and a recognizing portion connector is configured in the insertion portion so that an electrical connection is made.

6. The fractional laser surgical equipment of claim 5, wherein the recognizing portion recognizes one or more among a rotation quantity, a usage time, and a laser irradiation quantity of the insertion portion.

7. The fractional laser surgical equipment of claim 1, wherein the laser irradiation portion is a fixed module whose one side is fastened to the laser controller, in which a hollow portion, through which a laser emitted from the laser controller passes, is formed, and a focusing lens is configured in the hollow portion and which comprises guide portions at the other side of the laser irradiation portion.

8. The fractional laser surgical equipment of claim 7, wherein the guide portions having bar shapes comprise a pair of ⊏-shaped frames.
